Europäisches Patentamt

European Patent Office

Office européen des brevets

(19)

(11) Publication number: **0 151 419**

A2 ·

(12) **EUROPEAN PATENT APPLICATION**

(21) Application number: 85100522.3

(22) Date of filing: 18.01.85

(51) Int. Cl.⁴: **C 12 P 7/42**
//(C12P7/42, C12R1:72)

(30) Priority: 27.01.84 US 574507

(43) Date of publication of application:
14.08.85 Bulletin 85/33

(84) Designated Contracting States:
AT BE CH DE FR GB IT LI LU NL SE

(71) Applicant: E.R. Squibb & Sons, Inc.
Lawrenceville-Princeton Road
Princeton, N.J. 08540(US)

(72) Inventor: Robison, Robert S.
289 Clinton Rd.
North Brunswick New Jersey(US)

(72) Inventor: Szarka, Laszlo J.
5 Wellington Rd.
East Brunswick New Jersey(US)

(74) Representative: Vossius Vossius Tauchner Heunemann
Rauh
Siebertstrasse 4 P.O. Box 86 07 67
D-8000 München 86(DE)

(54) Method of preparing D(-)-beta-hydroxyisobutyric acid by fermentation.

(57) A method is provided for preparing D(−)-β-hydroxyisobutyric acid by fermentation employing isobutyryl chloride or the methyl and/or ethyl esters of isobutyric acid or the methyl and/or ethyl esters of methacrylic acid and/or the acetate and/or formate esters of isobutyl alcohol and/or isobutyl isobutyrate or isobutyl methacrylate as the substrate and a microorganism of the genus Candida and other fungi. In an alternative method, the isolated cells of the various microorganisms are employed with one or mixtures of the above substrates.

EP 0 151 419 A2

Our Ref.: T 500 EP
Case     : M-574,507-S

E.R. Squibb & Sons, Inc.
Princeton, New Jersey 08540
U.S.A.

# METHOD OF PREPARING D(-)-β-HYDROXYISOBUTYRIC ACID BY FERMENTATION

The present invention relates to a method for preparing D(-)-β-hydroxyisobutyric acid using as the substrate isobutyryl chloride, methyl or ethyl esters of isobutyric acid or methacrylic acid or esters of isobutyl alcohol.

In accordance with the present invention, there is provided a fermentation method for preparing the optically active D(-)-β-hydroxyisobutyric acid, which method includes the steps of subjecting a substrate, namely, isobutyryl chloride or the methyl and/or ethyl esters of isobutyric acid and/or the methyl and/or ethyl esters of methacrylic acid and/or esters of isobutyl alcohol to the action of a microorganism capable of converting one or more of the above substrates into D(-)-β-hydroxyisobutyric acid. The above reaction may be carried out in several different ways. In a preferred embodiment, the microorganism is cultivated aerobically in an aqueous nutrient medium containing all or a portion of the substrate so that cultivation of the microorganism and subjection of such portion of the substrate (present in the medium) to the action of the microorganism are carried out simultaneously. After a predetermined period and/or predetermined concentration of product is obtained, the remainder of the substrate, if any, is added, and the fermentation is continued until a desired concentration of product is obtained.

In alternative embodiments, the microorganism is first cultivated in an aqueous nutrient medium, then the substrate is added to the resulting culture broth or to a suspension of cells obtained through the initial cultivation, and the mixture is then incubated aerobically.

It has been found that in the method of the invention which employs as a substrate isobutyryl chloride and/or methyl and/or ethyl esters of isobutyric acid and/or methyl and/or ethyl esters of methacrylic acid and/or the esters of isobutyl alcohol, higher substrate concentrations may be used than in prior art fermentation techniques such as disclosed in U. S. Patent No. 4,310,635, so that higher product concentrations are obtainable in the method of the invention over prior art processes. For example, in the method of the invention, concentrations of substrate of more than about 4%, preferably more than about 3%, and optimally more than about 2% by weight of the combined culture medium (broth) and substrate volume are obtained leading to product concentrations of more than about 1.5% based on the volume of the broth, whereas in the fermentation techniques disclosed in U. S. Patent No. 4,310,635 substrate concentrations of up to about 3% by weight based on the combined volume of the culture broth and substrate are obtained leading to product concentrations of up to about 1% based on the total volume of broth and substrate.

In addition, it has been found that the solvent extraction technique employed in the method of the invention to recover hydroxylated products of the isobutyryl chloride and/or methyl and/or ethyl esters of isobutyric acid and/or methyl and/or ethyl esters of methacrylic acid and/or the esters of isobutyl alcohol is substantially more efficient than that employed to recover hydroxylated products of isobutyric acid, methacrylic acid, and isobutyl alcohol as disclosed in the prior art as discussed above.

The microorganism preferably employed in carrying out the method of the present invention will be of the genus _Candida_, and preferably of the strain _Candida rugosa_, for example, ATCC #20116. However, other microorganisms may be employed which may be selected by the screening procedure as disclosed in U. S. Patent No. 4,310,635 using isobutyryl chloride and/or methyl and/or ethyl esters of isobutyric acid and/or methyl and/or ethyl esters of methacrylic acid and/or the esters of isobutyl alcohol, such as isobutyl acetate, isobutyl formate and/or isobutyl-isobutyrate and/or isobutyl-methacrylate in place of isobutyric acid or methacrylic acid.

Examples of other microorganisms which may be employed herein belong to the genera Torulopsis, Trygonopsis, Saccharomyces, Pichia, Debaryomyces, Wingea, Rhodosporidium, Aspergillus, Choanephora, and Zygorhynchus. Among microorganisms belonging to the genera illustrated above, the following strains, for example, may be employed in the present invention: Candida parapsilosis, Candida utilis, Torulopsis candida, Trygonopsis variabilis, Saccharomyces cerevisiae, Saccharomyces rouxii, Pichia membranaefaciens, Debaryomyces hansenii, Wingea robertsii, Rhodosporidium toruloides, Aspergillus niger, Choanephora circinans and Zygorhynchus moelleri.

As indicated, for subjecting the substrate to the action of the microorganism, essentially two techniques are available. In a first technique, the microorganism is cultivated aerobically in an aqueous medium containing all or a portion of the substrate from the beginning of the cultivation, thereby accumulating $D(-)-\beta$-hydroxyisobutyric acid in the medium simultaneously with propagation of the microorganism. In the other technique, the process consists of two steps, that is, cultivation of the microorganism and subjection of the substrate to the action of the microorganism.

The first step of the two-step process can be carried out by cultivating the microorganism in an aqueous nutrient medium and recovering the cells. The second step of the two-step process can be carried out by adding the substrate to a suspension of the recovered cells or to immobilized cells in suitable aqueous buffer or medium followed by incubating the resulting mixture aerobically for all or part of the reaction time at a pH from about 6.0 to about 9.5 and at a temperature of from about 20 to about 40$^{o}$C.

Since enzymes of microorganisms are involved in the reaction of the process of the present invention, the separated cells can also be subjected to various treatments, such as drying and homogenization, etc., before suspension in an appropriate aqueous medium in order to promote the enzyme reaction. Therefore, use of cells treated in various ways should be construed as being covered by the scope of the present invention.

In carrying out the method wherein the microorganism is cultivated aerobically in an aqueous medium containing all or part of the substrate, reaction may be carried out by adding all or a portion of the substrate prior to growing the culture. The portion of substrate added to the culture prior to fermentation comprises from about 0.05 to about 0.2% and preferably from about 0.1 to about 0.15% by weight of the volume of aqueous medium to be used. The pH of the medium is adjusted, if necessary, to within the range of from about 4 to about 9.5, and preferably from about 6 to about 9, by adding strong inorganic base such as NaOH or KOH, while the reaction mixture is maintained at a temperature of from about 20 to about 40°C . The microorganism is cultivated aerobically and after a predetermined period of from about 12 to about 30 hours, and preferably from about 20 to about 25 hours, the remainder of the substrate is added. After addition of substrate is completed, the pH of the broth is adjusted within the range of from about 6 to about 9.5 and preferably from about 7 to about 8.5 by adding strong bases as described above. The fermentation is allowed to proceed for a total period of from about 40 to about 120 hours and preferably from about 48 to about 72 hours, until a concentration of product of from about 0.2 to about 1.5%, and preferably from about 0.5 to about 1.5% by volume of the broth is achieved.

Another embodiment of the method of the invention includes the steps of adding all of the substrate material after the microorganism is cultured in the culture medium to provide an initial broth concentration of substrate of within the range of from about 1 to about 4% by weight and preferably from about 2 to about 3% by weight based on the total volume of the culture medium or broth, maintaining the pH of the broth between from about 6 to about 9.5 and preferably from about 7 to about 8.5 by adding strong base such as KOH or NaOH, while maintaining the broth at a temperature of from about 20 to about 40$^O$C, and allowing fermentation to continue for a period within the range of from about 40 to about 120 hours and preferably from about 48 to about 72 hours until a peak concentration of product of from about 0.2 to about 1.5% and preferably from about 0.5 to about 1.5% by volume of the broth is achieved.

The fermentation media employed in the method of the invention includes a nitrogen source, a carbon/energy source, and optionally one or more inorganic salts for process control.

The nitrogen source will be present in an amount within the range of from about 0.1 to about 3%, and preferably from about 1 to about 2% by weight of the media. Examples of suitable nitrogen sources include casein hydrolysate, cottonseed or its derivatives, corn steep liquor, soybean meal, organic and inorganic compounds, such as $NH_4Cl$, $(NH_4)_2SO_4$, ammonia water, urea, amino acids, meat peptone and hydrolysates of soy bean meal or any other comparable organic or inorganic N sources or their soluble derivatives.

The carbohydrate source will be present in the fermentation media in an amount within the range of from about 0.5 to about 5% and preferably from about 1 to about 3% by weight. Examples of suitable carbohydrate sources include starch, dextrin, maltose, lactose, glucose, glycerol, molasses, and the like, organic acids, such as acetic acid, fumaric acid and lactic acid, alcohols such as methanol, ethanol and propanol, liquid hydrocarbons, such as n-paraffins and olefins, oils and fats and the like.

The fermentation media employed in the method of the invention may optionally include other conventional fermentation medium components such as one or more inorganic salts which aid in process control. Examples of such inorganic salts include, but are not limited to, $CaCO_3$, $CuSO_4$, NaCl, $ZnSO_4$, $FeSO_4$, $MgSO_4$, $MnSO_4$ or $Na_2HPO_4$ including hydrates thereof. The fermentation media may also contain one or more antifoam agents such as silicone antifoam.

A preferred fermentation medium formulation includes from about 1 to about 2% by weight of a nitrogen source, preferably a mixture of an organic and inorganic nitrogen, such as yeast extract and ammonium nitrate, from about 1 to about 3% by weight of glucose as the carbohydrate source, optionally from about 0.01% to about 1% by weight of one or more inorganic salts, such as potassium dihydrogen phosphate and magnesium sulfate, and optionally from about 0.01 to 0.2% by weight of a silicone antifoam.

The D(-)-β-hydroxyisobutyric acid product may be isolated from the culture broth or reaction mixture by subjecting the reaction mixture to filtration or centrifugation and then solvent extraction techniques. For example, in a preferred extraction method, the clarified culture broth or reaction mixture is acidified with a mineral acid, such as $H_2SO_4$ or HCl, to adjust pH to within the range of from about 1 to about 4 and preferably from about 2 to about 3. Inorganic salt such as $(NH_4)_2SO_4$ or $Na_2SO_4$ is added to increase ionic strength of the broth or mixture, and the mix is extracted with a water-immiscible solvent such as ethyl acetate, butanol or methyl isobutyl ketone. The desired product is obtained by distilling off the solvent and subjecting the residue to fractionation by, for example, vacuum distillation at a temperature in the range of 100-110$^{O}$C, or by column chromatography.

The following Examples represent preferred embodiments of the present invention. All temperatures are expressed in degrees Centigrade.

## Example 1
## Preparation of D(-)-β-Hydroxyisobutyric Acid Using Isobutyryl Chloride Substrate

10 Liters of the following medium were sterilized in a 14-liter glass fermentor after adjusting the pH of the medium to 7.5.

| Ingredient | Ingredient |
|---|---|
| Cerelose - 3.3% | $ZnSO_4 \cdot 7H_2O$ - 0.006% |
| Yeast Extract - 0.3% | $FeSO_4 \cdot 7H_2O$ - 0.009% |
| $(NH_4)_3PO_4$ - 1.3% | $CuSO_4 \cdot 5H_2O$ - 0.0005% |
| $KH_2PO_4$ - 0.7% | $MnSO_4 \cdot 4H_2O$ - 0.001% |
| $MgSO_4 \cdot 7H_2O$ - 0.08% | NaCl - 0.01% |
| Ucon Lubricant - 0.05% | |
| (silicone-type defoamer) | |

The glass fermentor was equipped with an agitator and sparger. The fermentor was operated at $30^{O}C$, and sterile air was supplied at 1 vol/vol medium/minute. The agitator speed was 500 rpm during the first 14 hours, and then 300 rpm until harvest. 100 ml of isobutyryl chloride was added after the medium had been sterilized and cooled to $30^{O}C$ and then the pH was adjusted to 7.3 with sodium hydroxide. 500 cc of a 24-hour broth culture of Candida rugosa ATCC #20116 was then used to inoculate the fermentor. After 23 hours, 100 ml of additional isobutyryl chloride was added, and the pH was adjusted to 8.35 with sodium hydroxide. At log 39, additional sodium hydroxide was added to adjust the pH to 8.5.

The synthesis of hydroxyisobutyric acid was followed by HPLC on clarified supernatants of the broth. After 43 hours of fermentation, the broth was found to contain 0.46% of hydroxyisobutyric acid. The hydroxyisobutyric acid was extracted with ethyl acetate after the clarified broth had been adjusted to a pH of 2.5 and saturated with ammonium sulfate. The ethyl acetate was removed by distillation. The oil residue was further purified by high vacuum distillation (0.2 mm) at 106-108°C. The optical rotation of the distillate fraction recovered after high vacuum distillation was found to be $[\alpha]_D^{23°}$ -14.6. This established that the hydroxyisobutyric acid synthesized with this ester of isobutyric acid had the desired D(-)-rotation while mass spectrometry indicated that the major component of this residue has a molecular weight of 104.

Example 2

Preparation of D(-)-β-hydroxyisobutyric Acid Using Ethyl Methacrylate Substrate

In this example, ethyl methacrylate was used as the substrate in place of isobutyryl chloride for the synthesis of D(-)-β-hydroxyisobutyric acid by Candida rugosa ATCC #20116.

The medium and operating conditions were essentially identical with those used in Example 1. All of the substrate, however, was added after 23 hours of fermentation to provide an initial broth concentration of ethyl methacrylate of 2.0%. After this substrate addition, the pH of the broth was maintained between 8.0 and 8.5 by the periodic addition of sodium hydroxide.

The synthesis of hydroxyisobutyric acid and the utilization of ethyl methacrylate was followed by HPLC. Hydroxyisobutyric acid was synthesized and reached a peak of 0.49% after 49 hours of fermentation.

Examples 3 to 9
Preparation of D(-)-β-Hydroxyisobutyric Acid Using Methyl Isobutyrate, Ethyl Isobutyrate, Methyl Methacrylate, Isobutyl Acetate, Isobutyl Formate, Isobutyl Isobutyrate or Isobutyl-Methacrylate as the Substrate

Following the procedure of Example 1 except substituting methyl isobutyrate (Example 3), ethyl isobutyrate (Example 4), methyl methacrylate (Example 5), isobutyl acetate (Example 6), isobutyl formate (Example 7), isobutyl-isobutyrate (Example 8) or isobutyl-methacrylate (Example 9) for isobutyl chloride, D(-)-β-hydroxyisobutyric acid is obtained.

HA303

0151419

## Example 10

Preparation of D(-)-β-Hydroxyisobutyric Acid
Wherein Methyl Isobutyrate is Added to a Cell
Suspension Obtained from a Fermentation Broth

Candida rugosa ATCC #20116 was inoculated into 20 L of a medium containing 2.0% glucose, 0.5% yeast extract, 0.3% peptone, 0.3% meat extract, 0.1% isobutyric acid and 0.05% Ucon Lubricant as a defoamer. The fermentation was conducted in a 38 L fermentor at 30°C, at a pH 6.0 and with 20 SLPM of air and agitation at 250 RPM. After 24 hours, the resulting cells, 5 g/liter of medium, were recovered by centrifugation, washed twice with 0.1% saline solution and suspended in 1/15 M pH 8.5 phosphate buffer.

To 100 ml of this cell suspension was added 3 g of methyl isobutyrate. The resulting mixture was incubated at 30°C and the synthesis of hydroxyisobutyric acid was followed by HPLC. After 40 hours of incubation, 0.4% of hydroxyisobutyric acid was synthesized.

## Examples 11 to 17

Preparation of D(-)-β-Hydroxyisobutyric Acid Using
Ethyl Isobutyrate, Methyl Methacrylate, Ethyl
Methacrylate, Isobutyl Acetate, Isobutyl Formate,
Isobutyl Isobutyrate or Isobutyl Methacrylate as
Substrates with Washed Cell uspensions of Candida
rugosa

Following the procedure of Example 10 except substituting ethyl isobutyrate (Example 11), methyl methacrylate (Example 12), ethyl methacrylate (Example 13), isobyutyl acetate (Example 14), isobyutyl formate (Example 15), isobutyl-isobutyrate (Example 16) or isobutyl methacrylate (Example 17) for methyl isobutyrate, D(-)-β-hydroxyisobutyric acid is obtained.

What is claimed is:

1.  A method for producing D(-)-β-hydroxyisobutyric acid, which comprises subjecting a substrate selected from the group consisting of isobutyryl chloride, the methyl ester of isobutyric acid, the ethyl ester of isobutyric acid, the methyl ester of methacrylic acid, the ethyl ester of methacrylic acid, one or more esters of isobutyl alcohol, and mixtures of two or more of the above, to the action of a microorganism having the ability to convert the substrate into D(-)-β-hydroxyisobutyric acid in an aqueous medium and recovering D(-)-β-hydroxyisobutyric acid from the medium.

2.  The method according to Claim 1 wherein the substrate is the acetate ester of isobutyl alcohol, the formate ester of isobutyl alcohol, isobutyl isobutyrate, isobutyl methacrylate or mixtures of two or more thereof.

3.  The method according to Claim 1 wherein the substrate is isobutyryl chloride, the methyl ester of isobutyric acid, the ethyl ester of isobutyric acid, the methyl ester of methacrylic acid, the ethyl ester of methacrylic acid or mixtures of two or more thereof.

4.  The method according to Claim 1 wherein said microorganism belongs to a genus selected from the group consisting of the genus Candida, Torulopsis, Trygonopsis, Saccharomyces, Pichia, Debaryomyces, Wingea, Rhodosporidium, Aspergillus, Choanephora, and Zygorhynchus.

HA303

0151419

5. The method according to Claim 1 wherein said microorganism belongs to the species <u>Candida</u> <u>rugosa</u>.

6. The method according to Claim 1 wherein the microorganism is cultivated aerobically in an aqueous medium containing the substrate.

7. The method according to Claim 6 wherein the cultivation is carried out at a pH of from about 4 to about 9.5 at a temperature of from about $20^{\circ}$C to about $40^{\circ}$C.

8. The method according to Claim 1 wherein the substrate is added to a culture broth obtained by cultivating the microorganism in an aqueous medium and then the resulting mixture is incubated aerobically.

9. The method according to Claim 8 wherein a small amount of said substrate is added in the aqueous medium when the microorganism is cultivated to induce enzymes of the microorganism.

10. The method according to Claim 6 wherein the microorganism is cultivated at a pH of from about 4.0 to about 9.5 and a temperature of from about $20^{\circ}$C to about $40^{\circ}$C and the mixture is incubated at a pH of from about 6.0 to about 9.5 and a temperature of from about $20^{\circ}$C to about $40^{\circ}$C.

11. The method according to Claim 1 wherein the substrate is added to a cell suspension prepared by separating cells from a culture broth obtained by cultivating the microorganism in an

HA303
0151419

aqueous medium followed by suspending the cells in an aqueous medium and then the resulting mixture is incubated aerobically for all or a part of the reaction time.

12. The method according to Claim 11 wherein the separated cells are immobilized on a suitable carrier before contact with the substrate.

13. The method according to Claim 11 wherein a small amount of said substrate is added in the aqueous medium when the microorganism is cultivated to induce the desired enzymes of the microorganism.

14. The method according to Claim 11 wherein the microorganism is cultivated at a pH of from about 4.0 to about 9.5 and a temperature of from about 20°C to about 40°C and the mixture is incubated at a pH of from about 6.0 to about 9.5 and a temperature of from about 20°C to about 40°C.

15. The method according to Claim 1 wherein D(-)-β-hydroxyisobutyric acid is recovered from the aqueous medium by extraction with a water-immiscible solvent.

16. The method according to Claim 15 wherein the water-immiscible solvent is butanol, methyl-isobutyl ketone, or ethyl acetate.